# EUROPÄISCHE PATENTSCHRIFT

(11) **EP 2 726 027 B1**
(45) Veröffentlichungstag und Bekanntmachung des Hinweises auf die Patenterteilung: **02.08.2017**
(21) Anmeldenummer: 12727783.8
(22) Anmeldetag: 15.05.2012
(51) Int. Cl.: A61F 2/91

(54) **KÖRPERIMPLANTAT MIT VERBESSERTER RÖNTGENSICHTBARKEIT UND VERFAHREN ZUM HERSTELLEN DESSELBEN**
BODY IMPLANT WITH IMPROVED X-RAY VISIBILITY, AND METHOD FOR PRODUCING SAME
IMPLANT CORPOREL PRÉSENTANT UNE VISIBILITÉ AUX RAYONS X AMÉLIORÉE ET PROCÉDÉ DE FABRICATION DUDIT IMPLANT

(30) Priorität: 28.09.2011 DE 102011115238
(43) Veröffentlichungstag der Anmeldung: 07.05.2014
(73) Patentinhaber: Admedes Schuessler GmbH, 75179 Pforzheim (DE)
(72) Erfinder: RASCHE, Ulrich, 76227 Karlsruhe (DE); KRAFT, Frank, 75242 Neuhausen (DE); HEGEL, Alexander, 76187 Karlsruhe (DE)
(74) Vertreter: Müller-Boré & Partner Patentanwälte PartG mbB
(86) Internationale Anmeldenummer: PCT/EP2012/002087
(87) Internationale Veröffentlichungsnummer: WO 2013/045000

(56) Entgegenhaltungen:
- EP-A2- 1 488 763
- WO-A1-97/33534
- US-A1- 2003 060 872
- US-A1- 2009 204 203

## Beschreibung

Die vorliegende Erfindung betrifft ein Körperimplantat, insbesondere einen Stent, zum Einführen in einen lebenden Körper mit einer guten Röntgensichtbarkeit sowie auf ein Verfahren zum Herstellen eines derartigen Körperimplantats.

Körperimplantate bzw. Stents dieser Art schützen Kanäle lebender Körper wie beispielsweise Blutgefäße, Speiseröhre, Harnröhre oder Nierengänge durch Einführen des zylindrischen Stents und Expandieren des Stents im Inneren des Körperkanals. Auf diese Weise kann ein Kollabieren oder Verschließen des jeweiligen Körperkanals verhindert werden. Darüber hinaus wird ein Stent beispielsweise für intercerebrale Gefäßaussackungen, sogenannte Aneurysmen eingesetzt, die die häufigste Ursache für nicht-traumatische Subarachnoidal-Blutungen sind. Ihre Inzidenz liegt in der Gesamtbevölkerung bei 1%, nach Autopsiestudien sogar bis zu 9%. Patomorphologisch sind intercerebrale Aneurysmen in der Regel echte, sacurale Aneurysmen, die meist in Gefäßaufzweigungen lokalisiert sind (vgl. beispielsweise Schumacher M. "Diagnostic work-up in cerebral aneurysms" in Nakstadt PHj (ed): "cerebral aneurysms", pp 13-24, Bologna: Centauro (2000)).

Darüber hinaus können derartige Körperimplantate bzw. Stents als Träger von Medikamenten dienen, um eine lokale Therapie innerhalb des Körperkanals zu ermöglichen.

Diese Stents werden in einem kollabierten Zustand in einen Körperkanal eingeführt und nach Anordnung des Stents in dem Körperkanal expandiert. Diese Stents bestehen üblicherweise aus rostfreiem Edelstahl oder einer Kobalt-Chrom-Tantal-Legierung. Die Stents werden bevorzugt durch eine Aufweitungseinrichtung wie beispielsweise einem Ballonkatheter in den Körperkanal eingeführt und dort aufgeweitet.

Die Stents können jedoch auch aus anderen Werkstoffen wie beispielsweise Polymeren, selbstabbaubaren Werkstoffen wie beispielsweise Milchsäure-Werkstoffen bzw. Derivaten, metallischen, bioabbaubaren Werkstoffen, wie beispielsweise Mg, Zink, Fe, sowie aus Nitinol (Nickel-Titan-Legierungen) und/oder aus anderen selbstexpandierbaren Werkstoffen wie beispielsweise sogenannten Formgedächtnis-Werkstoffen bestehen.

Um die Röntgensichtbarkeit von diesen Stents zu erhöhen, werden diese vielfach mit zusätzlichen Elementen (sogenannten Markern) versehen, die aus einem Material mit hoher Röntgensichtbarkeit hergestellt sind.

Bei sehr kleinen Stents, die beispielsweise im neuronalen Bereich eingesetzt werden, ist es schwierig, ausreichend sichtbare Röntgenmarker anzubringen. Es können Materialien mit einer höheren Dichte, wie beispielsweise Platin oder Platin-Iridium-Legierungen, angewandt werden, jedoch liegt deren Röntgensichtbarkeit lediglich etwa 7% über der von Gold. Darüber hinaus sind diese Materialien schwieriger zu bearbeiten.

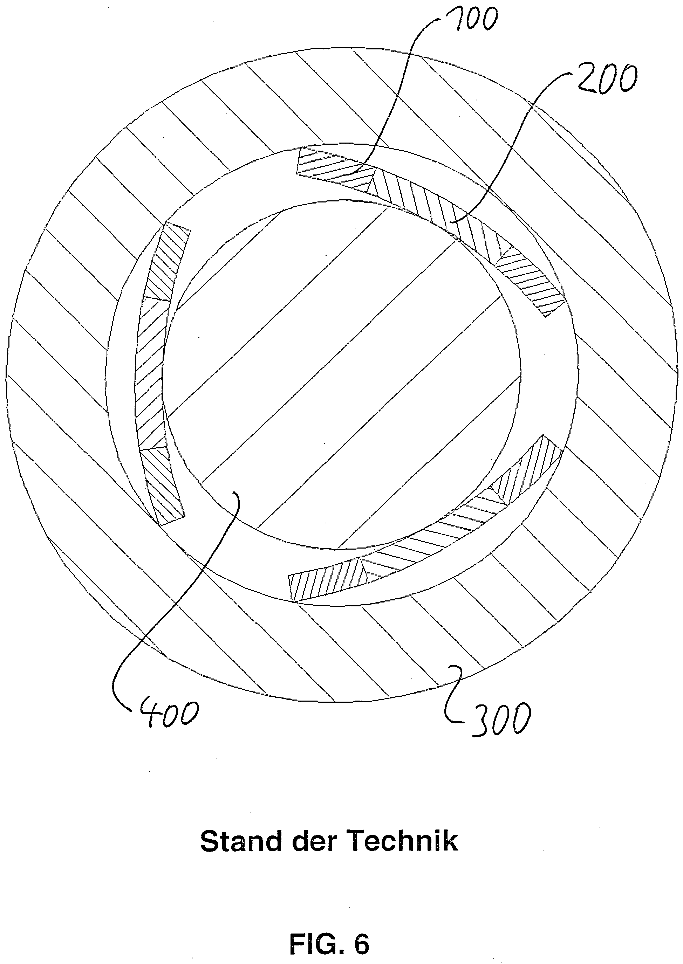

Wie in Fig. 6 gezeigt ist, ist ein bekanntes Markerelement 200 in einen Ausschnitt eines im wesentlichen zylindrischen Körperimplantats 100 eingesetzt und im wesentlichen bündig mit Rändern des Ausschnitts. Bei sehr kleinen Körperimplantaten, wie sie beispielsweise im neuronalen Bereich verwendet werden, wird das Markerelement 200 bei dieser Konfiguration jedoch so klein, daß die Röntgensichtbarkeit erschwert ist.

US 2009/0204203 A1 offenbart einen biologisch abbaubaren Stent mit röntgensichbaren Markern. WO 97/33534 A1 offenbart röntgensichtbare Stentmarker in der Gestalt von Nieten. EP 1 488 763 A2 beschreibt einen Stent mit einem daran angebrachten Hülsenmarker. US 2003/0060872 A1 beschreibt einen Stent mit röntgensichtbaren Eigenschaften.

Die Aufgabe der Erfindung besteht in der Schaffung eines Körperimplantats, das selbst bei geringen Abmessungen eine gute Röntgensichtbarkeit aufweist, das einfach und kostengünstig hergestellt werden kann, sowie eines Verfahrens zum Herstellen eines derartigen Körperimplantats und eines Verfahrens zum Ermöglichen bzw. Verbessern der Röntgensichtbarkeit eines Körperimplantats.

Diese Aufgabe wird durch ein Körperimplantat mit den Merkmalen der unabhängigen Ansprüche gelöst. Vorteilhafte Weiterbildungen der Erfindung sind in den abhängigen Ansprüchen definiert.

Gemäß einem Aspekt wird ein Körperimplantat zur Verfügung gestellt, insbesondere ein Stent, zum Einführen oder Implantieren in einen lebenden Körper mit einem Markerelement aus einem röntgensichtbaren Material, das in einen Ausschnitt des Körperimplantats eingesetzt ist, wobei das Markerelement auf zumindest einer Seite von einer Wandung des Ausschnitts des Körperimplantats zumindest teilweise hervorragt und der hervorragende Abschnitt des Markerelements eine Kreisbogenform aufweist.

In anderen Worten ist das Markerelement nicht bündig mit einer Oberfläche des Körperimplantats, sondern steht von dessen Oberfläche vor. Darüber hinaus ist der vorragende Abschnitt des Markerelements in der Kreisbogenform ausgebildet, um einerseits das Einsetzen eines Führungsdrahts oder Ballonkatheters in das Innere des Körperimplantats nicht zu behindern und andererseits das Einsetzen des Körperimplantats in einen Katheter nicht zu behindern.

Um eine gute Führung zwischen dem Markerelement und einem Außenumfang des Führungsdrahts bzw. Ballonkatheters zu gewährleisten und das Markerelement möglichst groß zu gestalten, hat der nach innen vorragende Abschnitt eine Kreisbogenform, die der Kreisbogenform des Außenumfangs des Führungsdrahts entspricht. Auf diese Weise wird erreicht, dass das Volumen zwischen Markerelement und Führungsdraht maximal ausgenützt wird. In anderen Worten wird ein Freiraum zwischen der Struktur und dem Führungsdraht genützt, um ein Volumen des Markerelements zu vergrößern, so dass selbst bei sehr kleinen Abmessungen des Körperimplantats eine ausreichende Röntgensichtbarkeit des Markerelements gewährleistet werden kann.

Der nach außen vorragende Abschnitt hat vorzugsweise auch eine Kreisbogenform, die in etwa mit der Kreisbogenform des Innendurchmessers eines Katheters übereinstimmt, mit dem das Körperimplantat in den lebenden Körper eingebracht wird. Dabei kann der außen vorragende Abschnitt an dem Innenumfang des Katheters bündig anliegen oder von diesem beabstandet sein. Es versteht sich, dass im Falle einer beabstandeten Konfiguration die Kreisbogenform des äußeren vorragenden Abschnitts von der Kreisbogenform des Katheters abweichen kann.

Vorzugsweise liegt ein Mittelpunkt der Kreisbogenform des hervorragenden Abschnitts innerhalb eines Querschnitts des Körperimplantats, vorzugsweise in der Nähe eines Mittelpunkts eines kreisförmigen Querschnitts des Körperimplantats, oder stimmt im wesentlichen mit diesem überein.

Weiter bevorzugt hat der Ausschnitt (Eyelet) eine Konusform oder Doppelkonusform zum Erzeugen eines Formschlusses mit dem Markerelement und/oder das Markerelement ist in den Ausschnitt eingepresst bzw. eingenietet.

Vorzugsweise ist die Kreisbogenform des Markers so konfiguriert, dass ein Führungsdraht beim Einführen in das Körperimplantat mit seiner Außenoberfläche durch die Kreisbogenform des Markers geführt werden kann.

Weiter bevorzugt ist die Kreisbogenform des Markers so konfiguriert, dass ein Katheter zum Einführen des Körperimplantats mit einem Durchmesser mit im wesentlichen dem Außendurchmesser des Körperimplantats mit seiner Innenoberfläche im wesentlichen bündig an der Kreisbogenform des Markers anliegen kann oder davon geringfügig beabstandet sein kann.

Vorzugsweise liegt ein Radius der Kreisbogenform des Markerelements im Bereich von etwa 0,8 Ri bis etwa 1,2 Ri, vorzugsweise im Bereich von etwa 0,9 Ri bis 1,1 Ri, wenn Ri den Radius des kontrahierten Körperimplantats darstellt. Ri ist dabei der halbe Innendurchmesser des kontrahierten Körperimplantats.

Vorzugsweise ist der hervorragende Abschnitt des Markerelements in Umfangsrichtung des Körperimplantats mit der Implantatstruktur überlappend.

Weiter bevorzugt weist das Markerelement ein Material mit hoher Absorption für Röntgenstrahlen wie beispielsweise Gold, Platin, Tantal, Platinlegierung, Platin-Iridium, Niob auf.

Gemäß einem weiteren Aspekt wird eine Kombination aus einem Katheter, einem Führungsdraht und einem Körperimplantat, wie zuvor beschrieben, zur Verfügung gestellt.

Gemäß einem weiteren Aspekt betrifft die Erfindung ein Verfahren zum Erhöhen bzw. Ermöglichen der Röntgensichtbarkeit eines Körperimplantats, insbesondere eines Stents, mit den Schritten:
Formen eines Ausschnitts in dem Körperimplantat mittels eines Lasers;
Einsetzen eines Markerelements in den Ausschnitt; und
Verpressen des Markerelements derart, dass das Markerelement auf zumindest einer Seite von einer Wandung des Ausschnitts zumindest teilweise hervorragt und der hervorragende Abschnitt des Markerelements eine Kreisbogenform aufweist, wobei ein nach innen vorragender Abschnitt der Kreisbogenform im wesentlichen mit einer Kreisbogenform eines Außenumfangs eines Führungsdrahts übereinstimmt.

Nachfolgend werden Ausführungsbeispiele der Erfindung anhand schematischer Zeichnungen näher erläutert.
Fig. 1 zeigt ein erstes Ausführungsbeispiel eines erfindungsgemäßen Körperimplantats im Querschnitt, das mittels eines Führungsdrahts in einen Katheter zum Einführen des Körperimplantats eingesetzt ist.
Fig. 2 zeigt ein zweites Ausführungsbeispiel des Körperimplantats ebenfalls im Querschnitt.
Fig. 3 zeigt ein drittes Ausführungsbeispiel des Körperimplantats im Querschnitt.
Fig. 4 zeigt in viertes Ausführungsbeispiel des Körperimplantats im Querschnitt.
Fig. 5 zeigt ein fünftes Ausführungsbeispiel des Körperimplantats im Querschnitt.
Fig. 6 zeigt ein Körperimplantat nach dem Stand der Technik im Querschnitt.
Fig. 7 zeigt eine Detailansicht von Fig. 1.

Nachfolgend werden Ausführungsbeispiele eines Körperimplantats 1 anhand eines Stents näher erläutert. Es versteht sich jedoch, dass die Erfindung nicht auf Stents beschränkt ist, sondern auch auf andere Körperimplantate angewandt werden kann, wie beispielsweise ein Blutflussmodifikator zur Beeinflussung des Blutstroms (Flow-Diverter) oder Thrombektomie-Geräte zum operativen Entfernen eines Blutgerinnsels aus einem Blutgefäß (Thrombectomy Devices), etc.

Ein Körperimplantat 1 bzw. Stent ist ein im wesentlichen zylindrisches Gebilde, das im kontrahierten Zustand in einen Einführ-Katheter 3 eingesetzt wird, um das Körperimplantat 1 in den lebenden Körper einzuführen. Aufgrund seiner Struktur, die aufweitbare Stege aufweist, kann der Stent nach dem Einführen und Plazieren aufgeweitet werden, um seine Gefäßstützfunktion zu erfüllen. Um den Stent am Röntgenschirm präzise zu plazieren, ist der Stent mit röntgensichtbaren Markern oder Markerelementen 2 versehen.

Die Herstellung des Stents kann sowohl aus Rohmaterial als auch aus Flachmaterial erfolgen, wobei bei letzterem der Stent später gerollt, geschweißt und/oder endbearbeitet wird. Weiterhin kann die Herstellung des Stents mittels Laserschneiden, Laserabtrag, photochemisches Ätzen und/oder Erodieren erfolgen. Weiterhin kann die Herstellung des Stents auch derart erfolgen, daß die Stentstruktur in einer zumindest teilweise expandierten Form gefertigt wird und der Stent anschließend zum Einführen z.B. in den Katheter auf eine komprimierte Form verkleinert wird, bevor er nachfolgend im Körper wieder zumindest teilweise expandiert wird.

Um das Körperimplantat 1 innerhalb des Einführ-Katheters 3 und darüber hinaus innerhalb des lebenden Körpers zu bewegen oder zu verlagern, ist das Körperimplantat 1 derart auf einen Führungs- oder Transportdraht 4 aufgeschoben, dass das Innenlumen des Körperimplantats 1 durch den Führungsdraht 4 im wesentlichen ausgefüllt ist. Auf diese Weise kann das Körperimplantat 1 entlang des Führungsdrahts 4 verlagert werden, um es an geeigneter Stelle im lebenden Körper zu plazieren.

Obwohl das Ausführungsbeispiel anhand des Führungs- oder Transportdrahts 4 beschrieben ist, kann statt dessen auch ein Ballonkatheter verwendet werden.

Das Körperimplantat 1 hat im gekrimmten, eingeführten Zustand einen Durchmesser von etwa 0,3 mm bis etwa 1,5 mm und im expandierten Zustand einen Durchmesser von etwa 1,5 mm bis etwa 6 mm. Um dieses Körperimplantat 1 einzuführen, wird ein Katheter 3 mit einer Größe von maximal 5 bis 6 French verwendet, der an seiner Spitze einen noch geringeren Durchmesser haben kann. Somit hat der Katheter 3 einen maximalen Aussendurchmesser von etwa 1,5 mm und einen Innendurchmesser von etwa 0,3 mm bis etwa 1,5 mm entsprechend dem Durchmesser des Körperimplantatsl im gekrimmten, eingeführten Zustand.

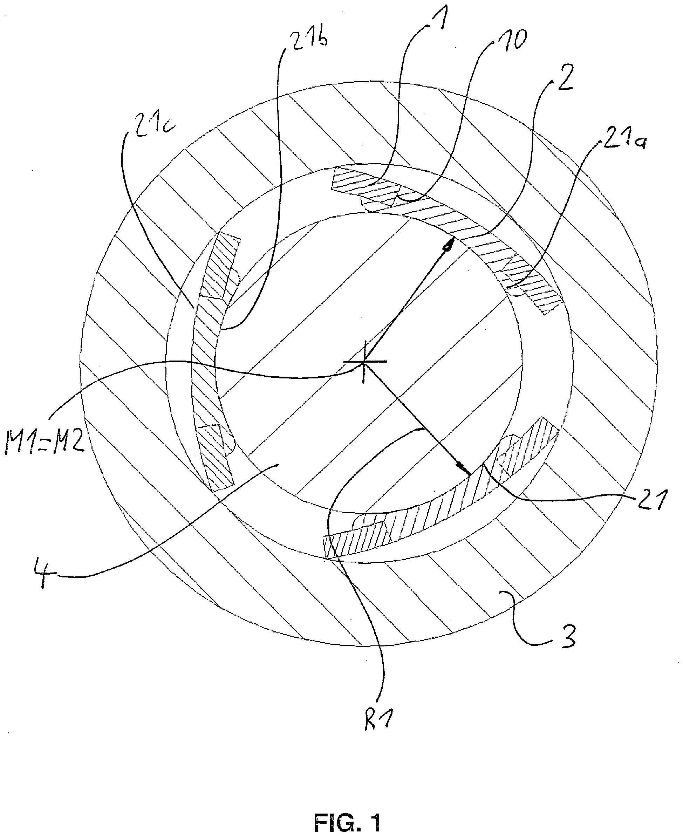

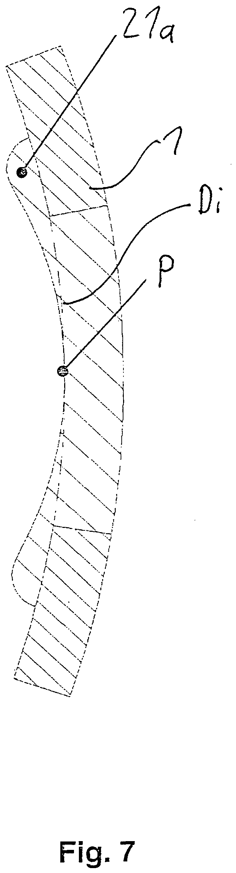

Wie in Fig. 1 und 7 gezeigt ist, ist ein Markerelement 2 in einen Ausschnitt 10 eines im wesentlichen zylindrischen Körperimplantats 1 eingesetzt. Der Ausschnitt 10, das sogenannte Eyelet, ist eine Öffnung in einem Steg des Körperimplantats 1, die vorzugsweise kreisrund ist. Vorzugsweise hat der Steg im Bereich des Ausschnitts 10 eine vergrößerte Breite, um eine Stegbreite im Bereich des Ausschnitts 10 nicht oder nur minimal zu verringern. Insbesondere bei den kleinen Körperimplantaten 1 wäre eine Verringerung der Stegbreite um die Breite des Ausschnitts 10 nachteilig, weil hierdurch die Festigkeit des Stegs beeinträchtigt wäre.

Der Ausschnitt 10 (=Eyelet) hat vorzugsweise einen Durchmesser von maximal 2 mm, vorzugsweise etwa 1 mm bis 1,5 mm. Wie ferner in Fig. 1 gezeigt ist, hat das Markerelement 2 des ersten Ausführungsbeispiels einen hervorragenden Abschnitt 21, der an einer Innenseite des Körperimplantats 1 von dem Ausschnitt 10 hervorragt. Auf diese Weise hat das Markerelement 2 eine vergrößerte Dicke, das heißt eine größere Wandstärke als das Körperimplantat 1. Um dabei das Einführen des Körperimplantats 1 mittels eines Führungsdrahts 4 und eines Katheters 3 nicht zu behindern, hat der hervorragende Abschnitt 21 im wesentlichen eine Kreisbogenform, die mit ihrer inneren Oberfläche 21 b im wesentlichen mit einem Außendurchmesser des Führungsdrahts 4 übereinstimmt. Auf diese Weise schmiegt sich die Kreisbogenform des hervorragenden Abschnitts 21 an den Führungsdraht 4 an, wenn das Körperimplantat 1 mittels eines Führungsdrahts 4 in einen Katheter 3 zum Einführen des Körperimplantats 1 in einen lebenden Körper eingesetzt ist.

Das Markerelement 2 kann an beliebiger Stelle im oder am Stent an dem sogenannten Eyelet (Ausschnittt 10) angebracht werden, beispielsweise am axialen Ende des Stents.

Um die Röntgensichtbarkeit weiter zu verbessern, hat das Markerelement 2 darüber hinaus einen überlappenden Abschnitt 21 a, der in Umfangsrichtung gegenüber dem Ausschnitt 10 überlappend ist. In anderen Worten hat der überlappende Abschnitt 21 a eine größere Ausdehnung als der Ausschnitt 10, so daß der überlappende Abschnitt 10 mit einer Wandung des Körperimplantats 1 überlappt.

In anderen Worten ist die Dicke des Markerelements 2 im Randbereich des Ausschnitts 10 und im Bereich des überlappenden Abschnitts 21 größer als in einem zentralen Bereich, d.h. einem Bereich in der Mitte des Ausschnitts 10. Vorzugsweise liegt der zentrale Bereich des Markerelements 2 mit dem Punkt P auf dem Innnendurchmesser Di des Körperimplantats 1, wie in Fig. 7 gezeigt ist.

Der überlappende Bereich 21 a steht gegenüber dem Innendurchmesser Di des Körperimplantats 1 um etwa 10% bis 60% der Wandstärke des Körperimplantats 1 vor, vorzugsweise um etwa 30% bis 40%.

Dabei wird der überlappende Bereich 21 a durch Nieten oder eine andere Form der plastischen Deformation, wie beispielsweise Laserbearbeitung, Schmelzprozesse oder Ultraschallschweißen hergestellt.

Somit ist das Markerelement 2 nicht bündig mit einer Oberfläche des Körperimplantats 1, sondern steht von dessen Oberfläche vor. Darüber hinaus ist der vorragende Abschnitt 21 des Markerelements 2 im wesentlichen in der Kreisbogenform ausgebildet, um einerseits das Einsetzen eines Führungsdrahts 4 in das Innere des Körperimplantats 1 nicht zu behindern und andererseits das Einsetzen des Körperimplantats 1 in einen Katheter 3 nicht zu behindern.

Um eine gute Führung zwischen dem Markerelement 2 und einem Außenumfang des Führungsdrahts 4 zu gewährleisten und das Markerelement 2 möglichst groß zu gestalten, hat der nach innen vorragende Abschnitt 21 vorzugsweise eine Kreisbogenform, die im wesentlichen der Kreisbogenform des Außenumfangs des Führungsdrahts 4 entspricht.

Der nach außen vorragende Abschnitt 21 hat vorzugsweise auch eine Kreisbogenform, die in etwa mit der Kreisbogenform des Innendurchmessers des Katheters 3 übereinstimmt, mit dem das Körperimplantat 1 in den lebenden Körper eingebracht wird. Dabei kann der außen vorragende Abschnitt 21 an dem Innenumfang des Katheters 3 bündig anliegen oder von diesem beabstandet sein.

Es versteht sich, dass im Falle einer beabstandeten Konfiguration die Kreisbogenform des äußeren vorragenden Abschnitts 21 von der Kreisbogenform des Katheters 3 abweichen kann.

Die Form des vorragenden Abschnitts 21 kann auch von der Kreisbogenform abweichen und beispielsweise flach gestaltet sein, so lange wie sich der vorragende Abschnitt 21 nicht mit dem Innendurchmesser des zu verwendenden Katheters 3 überschneidet. Es kommt lediglich darauf an, dass die Querschnittskontur des vorragenden Abschnitts 21 innerhalb einer durch die Querschnittsstruktur des Körperimplantats 1 definierten Hüllkurve liegt.

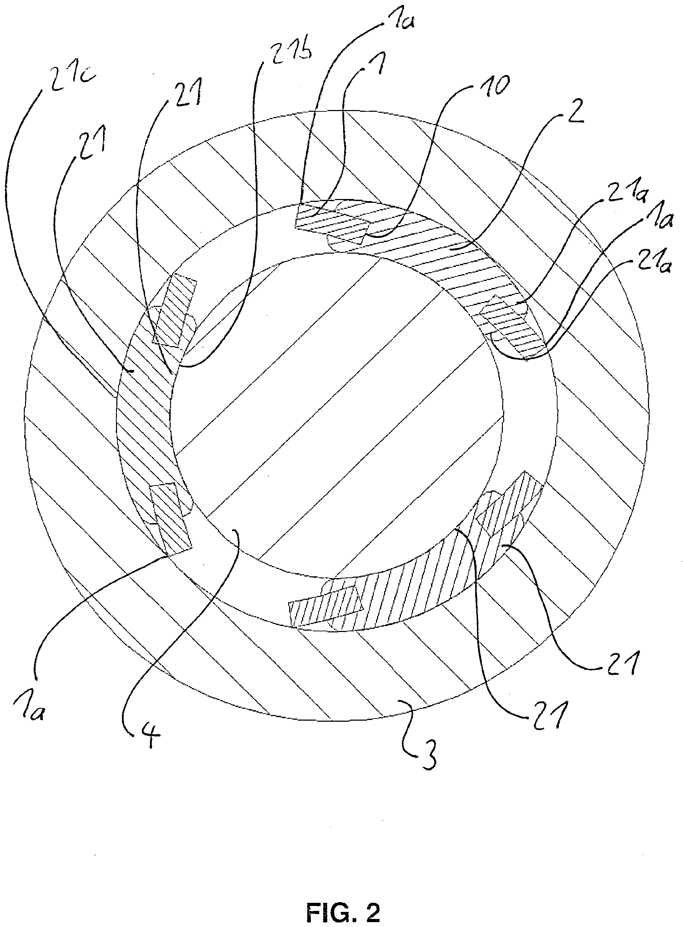

Fig. 2 zeigt ein zweites Ausführungsbeispiel. Dieselben oder ähnliche Elemente wie in dem ersten Ausführungsbeispiel werden mit denselben Bezugszeichen bezeichnet. Darüber hinaus werden dieselben Merkmale nicht erneut erläutert, um Wiederholungen zu vermeiden. Der Unterschied des zweiten Ausführungsbeispiels gegenüber dem ersten Ausführungsbeispiel liegt darin, daß der hervorragende Abschnitt 21 nicht nur an einer Innenseite des Körperimplantats 1, sondern zusätzlich auch an einer Außenseite des Körperimplantats 1 angeordnet ist. Derart kann eine Dicke des Markerelements 2 weiter vergrößert werden, um die Röntgensichtbarkeit zu verbessern. Dabei hat der hervorragende Abschnitt 21 a an der Außenseite des Körperimplantats 1 vorzugsweise eine Kreisbogenform, die sich beim Einsetzen in einen Katheter 3 mit ihrer äußeren Oberfläche 21 c an die Innenkontur des Katheters 3 anschmiegt bzw. bündig mit dieser ist. Auf diese Weise wird ein Einsetzen des Körperimplantats 1 in einen Katheter 3 nicht durch das dickere Markerelement 2 behindert.

Eine Ausdehnung in der radialen Richtung des äußeren hervorragenden Abschnitts 21 ist dabei so dimensioniert, dass der äußere hervorragende Abschnitt 21 innerhalb einer durch die Implantatstruktur bestimmten Hüllkurve liegt. Wie insbesondere in Fig. 2 gezeigt ist, definieren beispielsweise Randkanten oder - abschnitte 1 a des Körperimplantats 1 die obige Hüllkurve.

Darüber hinaus hat der auf der Außenseite des Körperimplantats 1 befindliche hervorragende Abschnitt 21 einen überlappenden Abschnitt 21 a, der die Wandung des Körperimplantats 1 überlappt. In anderen Worten hat der überlappende Abschnitt 21 a des hervorragenden Abschnitts 21 auf der Außenseite eine größere Erstreckung in Umfangsrichtung als der Ausschnitt 10.

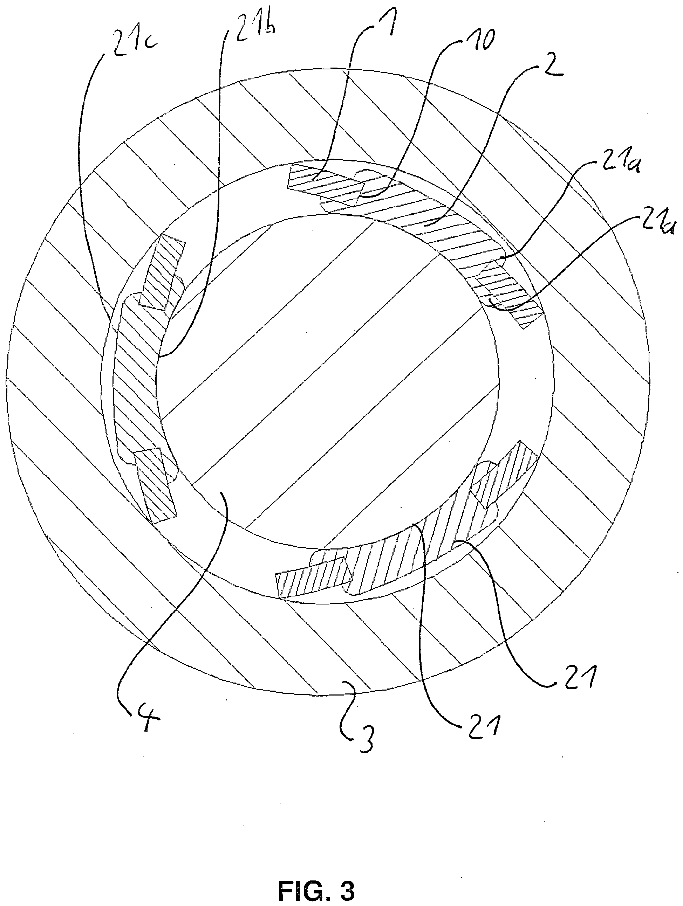

Fig. 3 zeigt ein drittes Ausführungsbeispiel. Dieses Ausführungsbeispiel ähnelt dem Ausführungsbeispiel von Fig. 2. Der hervorragende Abschnitt 21 a auf der Außenseite des Körperimplantats 1 ist jedoch in seiner Ausdehnung verringert. Insbesondere ist die Dicke des Markerelements 2 so gewählt, daß ein kleiner Abstand zwischen der Innenkontur des Katheters 3 und der äußeren Oberfläche 21c des hervorragenden Abschnitts 21 des Markerelements 2 beim Einführen des gekrimmten Körperimplantats 1 in den Katheter 3 vorgesehen ist. Auf diese Weise wird ein Einbringen des Körperimplantats 1 in den Katheter 3 erleichtert.

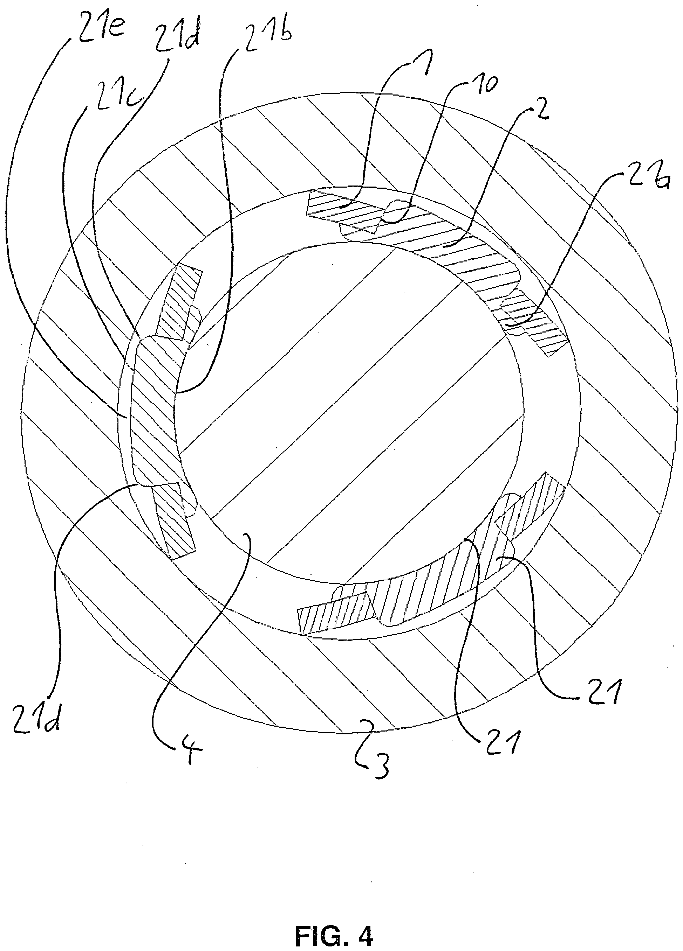

Fig. 4 zeigt ein viertes Ausführungsbeispiel der Erfindung. Dieses Ausführungsbeispiel ist ähnlich dem dritten Ausführungsbeispiel, das in Fig. 3 gezeigt ist, jedoch ist bei diesem Ausführungsbeispiel der überlappende Abschnitt 21 a auf der Außenseite des Körperimplantats 1 weggelassen. Auf diese Weise entsteht ein größerer Spalt zwischen der Innenkontur des Katheters 3 und der äußeren Oberfläche des Markerelements 2, so daß das Einführen des Körperimplantats 1 in den Katheter 3 weiter erleichtert ist. Insbesondere kann hierdurch der Spalt an den entgegengesetzten Randabschnitten 21 d des äußeren hervorragenden Abschnitts 21 vergrößert werden, um im wesentlichen dem Spalt im zentralen Abschnitt 21 e des hervorragenden Abschnitts 21 zu entsprechen. In anderen Worten liegt der Spalt an den entgegengesetzten Randabschnitten 21 d gegenüber dem im zentralen Abschnitt 21 e im Bereich von etwa 70% bis 130%, vorzugsweise im Bereich von etwa 85% bis 110%.

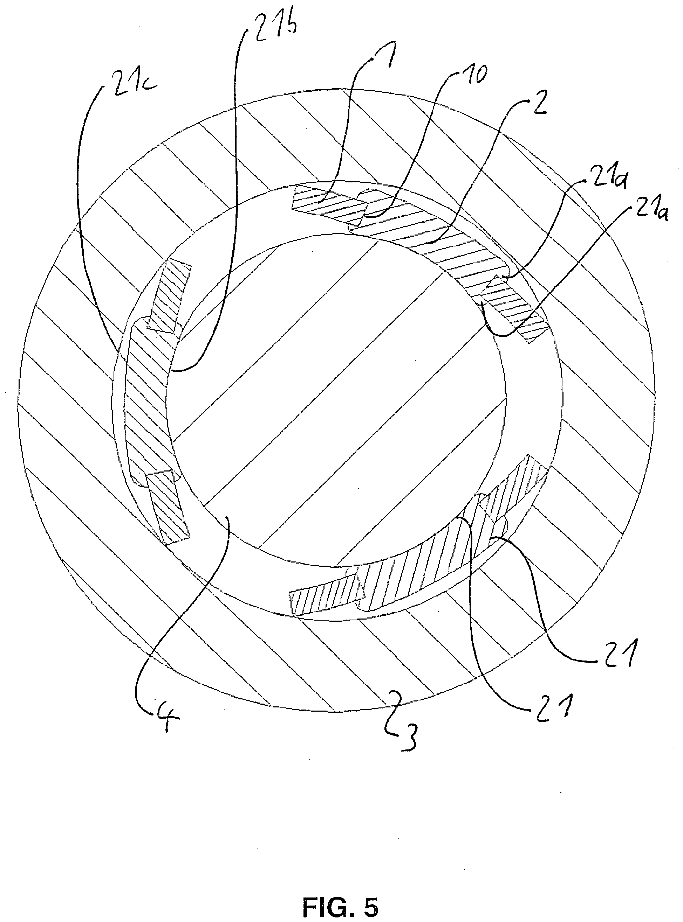

Fig. 5 zeigt ein fünftes Ausführungsbeispiel, der Erfindung. Dieses Ausführungsbeispiel hat hervorragende Abschnitte 21 a sowohl auf der Außen- als auch der Innenseite des Körperimplantats 1. Der hervorragende Abschnitt 21 a auf der Innenseite ist in einer Kreisbogenform derart gestaltet, daß das Markerelement 2 im kontrahierten Zustand des Körperimplantats 1 an einer Außenfläche des Führungsdrahts 4 im wesentlichen bündig anliegt. Im Gegensatz hierzu besteht ein kleiner Abstand zwischen dem hervorragenden Abschnitt 21 a auf der Außenseite und der Innenkontur des Katheters 3, um ein Einführen des Körperimplantats 1 in den Katheter 3 zu erleichtern. Ein überlappender Abschnitt 21 a ist ebenfalls sowohl auf der Innen- als auch auf der Außenseite vorgesehen, der überlappende Abschnitt 21 a ist jedoch insbesondere gegenüber dem zweiten Ausführungsbeispiel kleiner dimensioniert, so daß das Markerelement 2 einerseits sicher in dem Ausschnitt 10 fixiert ist, andererseits ein Einführen des Körperimplantats 1 in den Katheter 3 nicht behindert wird.

Bei allen Ausführungsbeispielen ist die Kreisbogenform des hervorragenden Abschnitts 21 so gestaltet, daß ein Mittelpunkt der Kreisbogenform innerhalb des Querschnitts des Körperimplantats 1 liegt. Besonders vorteilhaft ist es, wenn der Mittelpunkt M1 der Kreisbogenform im wesentlichen mit dem Mittelpunkt M2 des Querschnitts des Körperimplantats 1 übereinstimmt. Es ist jedoch nicht erforderlich, daß die Mittelpunkte M1 und M2 exakt übereinstimmen. Gewisse Abweichungen, um etwa 20 bis 30 Prozent der Länge eines Radius R1 der Kreisbogenform des hervorragenden Abschnitts 21 sind möglich. Auf diese Weise wird ein bündiges Anliegen der Kreisbogenform des Markerelements 2 an einem Führungsdraht 3 ermöglicht, um das Körperimplantat 1 in dem gekrimmten, eingeführten Zustand mit Hilfe des Führungsdrahts 3 zu führen oder zu transportieren bzw. zu verlagern.

In dem zweiten Ausführungsbeispiel ist die Kreisbogenform des äußeren hervorragenden Abschnitts 21 des Markerelements 2 bündig an dem Katheter 3 anliegend. Auf diese Weise wird ein Markerelement 2 mit maximaler Dicke vorgesehen, um eine gute Röntgensichtbarkeit insbesondere bei sehr kleinen Körperimplantaten 1 zu schaffen. Bei den anderen Ausführungsbeispielen ist das Markerelement 2 von einer Innenkontur des Katheters 3 beabstandet, um ein leichtes Einführen und Verschieben des Körperimplantats 1 innerhalb des Katheters 3 zu ermöglichen.

Der Mittelpunkt M1 der Kreisbogenform des äußeren hervorragenden Abschnitts 21 stimmt vorzugsweise ebenfalls im wesentlichen mit dem Mittelpunkt M2 des Körperimplantats überein oder ist um etwa 20 bis 30 Prozent der Länge des Radius R1 von dem Mittelpunkt M2 des Körperimplantats 1 versetzt angeordnet. Auf diese Weise wird ein Markerelement 2 mit einer verhältnismäßig großen Dicke vorgesehen, ohne eine Verschiebbarkeit oder Beweglichkeit des Körperimplantats 1 innerhalb des Katheters 3 zu behindern.

Obwohl der äußere hervorragende Abschnitt 21 die Kreisbogenform hat, kann die äußere Oberfläche 21 c des Markerelements 2 in den Ausführungsbeispielen der Figuren 1, 3, 4 und 5 auch eine im wesentlichen flache Oberfläche aufweisen, weil in diesen Ausführungsbeispielen die äußere Oberfläche 21 c nicht an der Innenkontur des Katheters 3 anliegt.

Der Marker 2 weist vorzugsweise ein röntgensichtbares Material, wie beispielsweise Gold, Platin, Tantal, Niob oder eine Platinlegierung, wie beispielsweise Platin-Iridium, auf.

Das Körperimplantat 1 weist bevorzugt eines der folgenden Materialien auf: rostfreier Edelstahl, Kobalt-Chrom-Tantal-Legierung, Polymere, selbstabbaubare Werkstoffe wie beispielsweise Milchsäure-Werkstoffe bzw. Derivaten sowie Nitinol (Nickel-Titan-Legierungen) und/oder andere selbstexpandierbare Werkstoffe wie beispielsweise sogenannten Formgedächtnis-Werkstoffen.

### Bezugszeichenliste

- 1: Körperimplantat
- 1a: Randabschnitt
- 2: Markerelement
- 3: Katheter
- 4: Führungsdraht
- 10: Ausschnitt
- 11: Implantatstruktur
- 21: hervorragender Abschnitt
- 21a: überlappender Abschnitt
- 21b: innere Oberfläche
- 21c: äußere Oberfläche
- 21d: Randabschnitt
- 21e: zentraler Abschnitt

- Di: Innendurchmesser des Körperimplantats
- M1: Mittelpunkt der Kreisbogenform
- M2: Mittelpunkt des Körperimplantats
- R1: Radius der Kreisbogenform

## Patentansprüche

1. Körperimplantat (1), insbesondere Stent, zum Einführen oder Implantieren in einen lebenden Körper mit einem Markerelement (2) aus einem röntgensichtbaren Material, das in einen Ausschnitt (10) des Körperimplantats (1) eingesetzt ist, wobei das Markerelement (2) auf zumindest einer Seite von einer Wandung des Ausschnitts (10) des Körperimplantats (1) zumindest teilweise hervorragt und der hervorragende Abschnitt (21) des Markerelements (2) eine Kreisbogenform aufweist, wobei ein nach innen vorragender Abschnitt der Kreisbogenform im wesentlichen mit einer Kreisbogenform eines Außenumfangs eines Führungsdrahts (4) übereinstimmt.

2. Körperimplantat (1) nach Anspruch 1, wobei ein Mittelpunkt (M1) der Kreisbogenform des hervorragenden Abschnitts innerhalb eines Querschnitts des Körperimplantats (1) liegt, vorzugsweise in der Nähe eines Mittelpunkts (M2) eines kreisförmigen Querschnitts des Körperimplantats (1) liegt oder im wesentlichen mit diesem übereinstimmt.

3. Körperimplantat (1) nach einem der vorherigen Ansprüche, wobei der Ausschnitt (10) eine Konusform oder Doppelkonusform zum Erzeugen eines Formschlusses mit dem Markerelement (2) hat und/oder das Markerelement (2) in den Ausschnitt (10) eingepresst bzw. eingenietet ist.

4. Körperimplantat (1) nach einem der vorherigen Ansprüche, wobei die Kreisbogenform des Markers (2) so konfiguriert ist, dass ein Katheter (3) zum Einführen des Körperimplantats (1) mit einem Durchmesser (Dk) mit im wesentlichen dem Außendurchmesser (Da) des Körperimplantats (1) mit seiner Innenoberfläche im wesentlichen bündig an der Kreisbogenform des Markers (2) anliegen kann oder davon geringfügig beabstandet sein kann.

5. Körperimplantat (1) nach einem der vorherigen Ansprüche, wobei ein Radius (R1) der Kreisbogenform des Markerelements (2) im Bereich von etwa 0,8 Ri bis etwa 1,2 Ri liegt, vorzugsweise im Bereich von etwa 0,9 Ri bis 1,1 Ri, wenn Ri den Radius des kontrahierten Körperimplantats (1) darstellt.

6. Körperimplantat (1) nach einem der vorherigen Ansprüche, wobei der hervorragende Abschnitt (21) des Markerelements (2) in Umfangsrichtung des Körperimplantats (1) mit der Implantatstruktur überlappend ist.

7. Körperimplantat (1) nach einem der vorherigen Ansprüche, wobei das Markerelement (2) ein Material mit hoher Absorption für Röntgenstrahlen wie beispielsweise Gold, Platin, Tantal, Platinlegierung, Platin-Iridium, Niob aufweist.

8. Kombination aus einem Katheter (3), einem Führungsdraht (4) und einem Körperimplantat(1) nach einem der vorherigen Ansprüche.

9. Verfahren zum Erhöhen bzw. Ermöglichen der Röntgensichtbarkeit eines Körperimplantats (1), insbesondere eines Stents, mit den Schritten:
Formen eines Ausschnitts (10) in dem Körperimplantat (1) mittels eines Lasers;
Einsetzen eines Markerelements (2) in den Ausschnitt (10); und
Verpressen des Markerelements (2) derart, dass das Markerelement (2) auf zumindest einer Seite von einer Wandung des Ausschnitts (10) zumindest teilweise hervorragt und der hervorragende Abschnitt (21) des Markerelements (2) eine Kreisbogenform aufweist, wobei ein nach innen vorragender Abschnitt der Kreisbogenform im wesentlichen mit einer Kreisbogenform eines Außenumfangs eines Führungsdrahts (4) übereinstimmt.

## Claims

1. A body implant (1), in particular a stent, for inserting or implanting in a living body with a marker element (2) made of a radiopaque material, which is inserted into a cutout (10) of the body implant (1), wherein said marker element (2) on at least one side protrudes from a wall of the cutout (10) of the body implant (1) at least partially and the protruding portion (21) of the marker element (2) has a circular arc shape, wherein an inward protruding portion of the circular arc shape substantially corresponds to a circular arc shape of an outer diameter of a guide wire (4).

2. The body implant (1) according to claim 1, wherein a center point (M1) of the circular arc shape of the protruding portion is within a cross section of the body implant (1), preferably is near a center point (M2) of a circular cross section of the body implant (1) or substantially corresponds thereto.

3. The body implant (1) according to one of the preceding claims, wherein the cutout (10) has a cone shape or double cone shape to create a positive locking with the marker element (2) and/or the marker element (2) is pressed or riveted into the cutout (10).

4. The body implant (1) according to one of the preceding claims, wherein the circular arc shape of the marker (2) is configured such that a catheter (3) for introducing the body implant (1) with a diameter (Dk) having substantially the outer diameter (Da) of the body implant (1) can abut the circular arc shape of the marker (2) in a substantially flush way with its inner surface or can be slightly spaced therefrom.

5. The body implant (1) according to one of the preceding claims, wherein a radius (R1) of the circular arc shape of the marker element (2) is in the range of about 0.8 Ri to about 1.2 Ri, preferably in the range of about 0.9 Ri to 1.1 Ri, when Ri represents the radius of the contracted implant body (1).

6. The body implant (1) according to one of the preceding claims, wherein the protruding portion (21) of the marker element (2) overlaps with the implant structure in the circumferential direction of the body implant (1).

7. The body implant, (1) according to one of the preceding claims, wherein the marker element (2) comprises a material with high absorption for X-rays, such as gold, platinum, tantalum, platinum alloy, platinum-iridium, niobium.

8. A combination of a catheter (3), a guide wire (4), and a body implant (1) according to one of the preceding claims.

9. A method for increasing or allowing the radiopaque visibility of a body implant (1), in particular a stent, comprising the steps of:
forming a cutout (10) in the body implant (1) by means of a laser;
inserting a marker element (2) in the cutout (10); and
compressing the marker element (2) such that the marker element (2) on at least one side protrudes from a wall of the cutout (10) at least partially and the protruding portion (21) of the marker element (2) has a circular arc shape, wherein an inward protruding portion of the circular arc shape substantially corresponds to a circular arc shape of an outer diameter of a guide wire (4).

## Revendications

1. Implant corporel (1), notamment stent, pour l'introduction ou l'implantation dans un corps vivant avec un élément marqueur (2) en un matériau visible aux rayons X qui est inséré dans une découpe (10) de l'implant corporel (1), dans lequel l'élément marqueur (2) fait au moins partiellement saillie sur au moins un côté depuis une paroi de la découpe (10) de l'implant corporel (1) et la section saillante (21) de l'élément marqueur (2) présente une forme d'arc de cercle, dans lequel une section dépassant vers l'intérieur de la forme d'arc de cercle coïncide essentiellement avec une forme d'arc de cercle d'une périphérie externe d'un fil de guidage (4).

2. Implant corporel (1) selon la revendication 1, dans lequel un centre (M1) de la forme d'arc de cercle de la section saillante se situe au sein d'une section transversale de l'implant corporel (1), se situe de préférence à proximité d'un centre (M2) d'une section transversale circulaire de l'implant corporel (1) ou coïncide essentiellement avec celui-ci.

3. Implant corporel (1) selon une des revendications précédentes, dans lequel la découpe (10) a une forme de cône ou forme de double cône pour la génération d'une conjugaison de formes avec l'élément marqueur (2) et/ou l'élément marqueur (2) est enfoncé ou riveté dans la découpe (10).

4. Implant corporel (1) selon une des revendications précédentes, dans lequel la forme d'arc de cercle du marqueur (2) est configurée de sorte qu'un cathéter (3) pour l'introduction de l'implant corporel (1) avec un diamètre (Dk) avec essentiellement le diamètre externe (Da) de l'implant corporel (1) peut reposer avec sa surface interne essentiellement à fleur sur la forme d'arc de cercle du marqueur (2) ou peut être faiblement écarté de celle-ci.

5. Implant corporel (1) selon une des revendications précédentes, dans lequel un rayon (R1) de la forme d'arc de cercle de l'élément marqueur (2) se situe dans la région d'environ 0,8 Ri à environ 1,2 Ri, de préférence dans la région d'environ 0,9 Ri à environ 1,1, Ri, lorsque Ri représente le rayon de l'implant corporel contracté (1).

6. Implant corporel (1) selon une des revendications précédentes, dans lequel la section saillante (21) de l'élément marqueur (2) est chevauchante avec la structure d'implant dans la direction périphérique de l'implant corporel (1).

7. Implant corporel (1) selon une des revendications précédentes, dans lequel l'élément marqueur (2) présente un matériau à haute absorption aux rayons X comme par exemple de l'or, du platine, du tantale, un alliage de platine, du platine-iridium, du niobium.

8. Combinaison d'un cathéter (3), d'un fil de guidage (4) et d'un implant corporel (1) selon une des revendications précédentes.

9. Procédé pour augmenter ou rendre possible la visibilité aux rayons X d'un implant corporel (1), notamment d'un stent, avec les étapes :
façonnage d'une découpe (10) dans l'implant corporel (1) au moyen d'un laser ; insertion d'un élément marqueur (2) dans la découpe (10) ; et
compression de l'élément marqueur (2) de telle sorte que l'élément marqueur (2) fait au moins partiellement saillie sur au moins un côté depuis une paroi de la découpe (10) et la section saillante (21) de l'élément marqueur (2) présente une forme d'arc de cercle, dans lequel une section dépassant vers l'intérieur de la forme d'arc de cercle coïncide essentiellement avec une forme d'arc de cercle d'une périphérie externe d'un fil de guidage (4).
